(19) Europäisches Patentamt
European Patent Office
Office européen des brevets

(11) EP 0 792 338 B1

(12) EUROPÄISCHE PATENTSCHRIFT

(45) Veröffentlichungstag und Bekanntmachung des Hinweises auf die Patenterteilung:
**10.03.1999 Patentblatt 1999/10**

(21) Anmeldenummer: **95939242.4**

(22) Anmeldetag: **08.11.1995**

(51) Int Cl.[6]: **C11D 1/835**, C11D 1/65, C11D 3/18, C11D 3/20, D06M 13/463, D06M 13/224, D06M 13/02, A61K 7/50

(86) Internationale Anmeldenummer:
**PCT/EP95/04397**

(87) Internationale Veröffentlichungsnummer:
**WO 96/16147 (30.05.1996 Gazette 1996/25)**

(54) **KATIONISCHE WACHSDISPERSIONEN**

CATIONIC WAX DISPERSIONS

DISPERSIONS AQUEUSES DE CIRE

(84) Benannte Vertragsstaaten:
**AT BE DE ES FR IT NL**

(30) Priorität: **17.11.1994 DE 4441029**

(43) Veröffentlichungstag der Anmeldung:
**03.09.1997 Patentblatt 1997/36**

(73) Patentinhaber: **Henkel Kommanditgesellschaft auf Aktien**
**40191 Düsseldorf (DE)**

(72) Erfinder:
- **GUCKENBIEHL, Bernhard**
**D-40211 Düsseldorf (DE)**
- **FÖRSTER, Thomas**
**D-40699 Erkrath (DE)**
- **HENSEN, Hermann**
**D-42781 Haan (DE)**
- **WILSCH-IRRGANG, Anneliese**
**D-45549 Sprockhövel (DE)**
- **KAHRE, Jörg**
**D-40789 Monheim (DE)**

(56) Entgegenhaltungen:
**EP-A- 0 082 456**
**WO-A-93/18222**
**WO-A-94/27570**
**DE-A- 2 830 173**
**FR-A- 2 352 046**
**FR-A- 2 398 832**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft feindisperse und stabile Dispersionen wachsartiger Fettstoffe und Paraffine mit einem Gehalt an kationischen Tensiden und wasserlöslichen Elektrolytsalzen.

Aus der deutschen Patentanmeldung DE 38 19 193 AI ist ein Verfahren zur Herstellung niedrigviskoser Öl-in-Wasser-Emulsionen bekannt, bei welchem nichtionogene Emulgatoren eingesetzt werden und die Emulsion auf Temperaturen oberhalb der Phaseninversionstemperatur erwärmt oder bei dieser Temperatur hergestellt wird. Es wurde weiterhin bereits festgestellt, daß dieses Verfahren auch auf Wachse angewandt werden kann. Schließlich betrifft die noch unveröffentlichte deutsche Patentanmeldung P 43 18 171.6 wäßrige Zubereitungen ionischer Tenside, die solche feindisperse Emulsionen oder Dispersionen enthalten und aufgrund ihres Gehalts an feindispersen Ölen und Wachsen besondere anwendungstechnische Eigenschaften aufweisen.

Aus FR-A-2 398 832, FR-A-2 352 046 und WO-A-93/18222 waren Paraffin- und Wachs-Dispersionen bekannt, die nichtionogene und kationische Emulgatoren, aber keine wasserlöslichen Alkali- oder Erdalkalisalze, enthalten und deren Teilchendurchmesser nicht angegeben ist.

Besonders den Zubereitungen kationischer Tenside kommt in der Textiltechnik. in der Haut- und Haarkosmetik und auf dem Gebiet der Wäscheweichspülmittel eine besondere Bedeutung zu. Ein Gehalt an feindispergierten Wachsen in solchen kationischen Tensiden führt z.B. zu einer erheblichen Verstärkung der antistatischen, avivierenden und faserweichmachenden Effekte, vermutlich infolge einer verstärkten Adsorption der dispergierten Wachse an die Faser.

Es hat sich aber gezeigt, daß der Gehalt ionischer Tenside die Phaseninversionstemperatur auf Werte über 100°C erhöhen kann, so daß solche Dispersionen ohne Anwendung von Druck nicht in der erwünschten Feinteiligkeit herstellbar sind.

Es bestand daher die Aufgabe, feinteilige Dispersionen von wachsartigen Fettstoffen und Paraffinen mit einem Gehalt an kationischen Tensiden ohne Anwendung von erhöhtem Druck herzustellen.

Es wurde gefunden, daß die Phaseninversionstemperatur durch einen Zusatz wasserlöslicher Elektrolyte zur wäßrigen Phase auf Werte unter 100°C gesenkt werden kann und daher die Herstellung der besonders feinteiligen Dispersionen ohne Anwendung von Druck möglich wird.

Gegenstand der Erfindung sind daher wäßrige Dispersionen wachsartiger Fettstoffe und Paraffine mit einem mittleren Teilchendurchmesser von 100 bis 500 nm, die

(A) 20 bis 50 Gew.-% dispergierte wachsartige bei Normaltemperatur (20° C) feste und in einem Bereich von 40 - 90° C schmelzbare Fettstoffe oder Paraffine

(B) 1 bis 15 Gew.-% nichtionogene Emulgatoren

(C) 0,5 bis 40 Gew.-% kationische Tenside und

(D) 1 bis 5 Gew.-% wasserlösliche Elektrolytsalze, die bei Normaltemperatur (20°C) zu wenigstens 1 Gew.-% in der wäßrigen Phase gelöst sind,

enthalten.

Bei den erfindungsgemäßen Dispersionen handelt es sich weder um optisch isotrope einphasige Systeme, wie sie bei Solubilisaten und Mikroemulsionen vorliegen, deren Teilchendurchmesser weit unter 100 nm liegt, noch handelt es sich um milchig weiße Emulsionen, deren Teilchendurchmesser um 1000 nm beträgt.

Die erfindungsgemäßen PIT-Dispersionen zeigen vielmehr aufgrund ihrer Teilchengröße ein in der Durchsicht braunrot und im Auflicht bläulich schimmerndes Aussehen, das durch die Tyndall-Streuung an den Emulsionströpfchen erklärt wird.

Unter wachsartigen Fettstoffen und Paraffinen sollen bei Normaltemperatur (20°C) feste und in einem Bereich von 40 - 90°C schmelzbare, wasserunlösliche Fette, Wachse und Kohlenwasserstoffe verstanden werden. Solche Fettstoffe sind z.B. gehärtete tierische Fette und pflanzliche Öle und synthetische Triglyceride von Fettsäuren mit 12 - 22 C-Atomen, Fettsäure-Fettalkohol-Ester aus gesättigten $C_{12}$-$C_{22}$-Fettsäuren und $C_{12}$-$C_{22}$-Fettalkoholen und andere aliphatische Ester im genannten Schmelzbereich. Bevorzugt geeignete Fettstoffe sind Ester aus Fettsäuren mit 12 - 22 C-Atomen und Fettalkohole mit 12 - 22 C-Atomen, z.B. Cetylpalmitat, Stearylstearat und Behenylstearat und Sterine, z.B. Sojasterine.

Geeignete nichtionogene Emulgatoren bestehen bevorzugt aus einer Kombination von

(B1) einem hydrophilen nichtionischen Emulgator mit einem HLB-Wert von 11 bis 15 und

(B2) einen lipophilen Coemulgator aus der Gruppe der gesättigten Fettalkohole mit 16 bis 22 C-Atomen und Partialestern von Polyolen mit 2 - 6 C-Atomen und Fettsäuren mit 16 - 22 C-Atomen.

Diese liegen bevorzugt in einem Gewichtsverhältnis von (B1) : (B2) = 1 : 1 bis 5 : 1 vor.

Bei den hydrophilen Emulgatoren handelt es sich bevorzugt im Ethylenoxid-Anlagerungsprodukte an Fettalkohole mit 16 - 22 C-Atomen oder an Partialester von Polyolen mit 3 - 6 C-Atomen und Fettsäuren mit 14 - 22 C-Atomen. Geeignet sind aber auch Ethylenoxidanlagerungsprodukte an Fettsäuren, an Alkylglucoside, an Methylglucosid-Fettsäureester, an Fettsäurealkanolamide, an Fettsäure-glucamide und andere Fettstoffe mit ethoxylierbaren Substituenten. Es kann besonders bevorzugt sein, als hydrophile Emulgatoren Alkylpolyglycoside der Formel $R0 - (Z)_x$ einzusetzen, in der R einen $C_{8-22}$-Alkyl- oder -Alkenylrest, Z einen Monosaccharid, insbesondere Glucose, und x eine Zahl von 1,1 bis 5, insbesondere von 1,2 bis 1,6 darstellt.

Bei den lipophilen Coemulgatoren handelt es sich bevorzugt um gesättigte Fettalkohole mit 16 - 22 C-Atomen oder freie Fettsäuren mit 16 - 22 C-Atomen, um Partialester von Polyolen mit 3 - 6 C-Atomen mit gesättigten Fettsäuren mit 14 - 22 C-Atomen, z.B. Glycerin- oder Sorbitan-monofettsäureester, um Glycolmonofettsäureester, um Fettsäurealkanolamide aus $C_{12}$-$C_{18}$-Fettsäuren mit Mono- oder Dialkanolaminen mit 2 - 4 C-Atomen in der Alkanolgruppe oder um Glycerin-mono-fettalkoholether.

Unter dem HLB-Wert soll dabei eine Größe verstanden werden, die aus der Struktur des Emulgators errechnet werden kann gemäß

$$HLB = \frac{100 - L}{5}$$

worin L der Gewichtsanteil (in %) der lipophilen Gruppen, z.B. der Fettalkyl- bzw. Fettacyl-gruppen im Emulgator ist.

Als kationische Tenside (C) eignen sich Verbindungen, die wenigstens eine lipophile Alkyl- oder Acylgruppe, bevorzugt mit 10 - 22 C-Atomen und eine protonierbare Aminogruppe oder eine quartäre Ammoniumgruppe aufweisen. Die Amino- oder Ammoniumgruppe kann dabei auch Teil eines heterocyclischen Rings, z.B. eines Pyridin- oder Imidazolinrings, sein. Die kationischen Tenside mit einer lipophilen Gruppe sind dabei in Wasser leicht löslich, sie liegen also in den erfindungsgemäßen Zubereitungen in der wäßrigen Phase gelöst vor. Kationische Tenside mit mehr als einer lipophilen Alkyl- oder Acylgruppe sind nur begrenzt wasserlöslich und bilden einen Teil der dispersen Phase. Sie verfügen aber über ein besonders starkes Aufziehvermögen auf Grenzflächen, insbesondere auf Haare und textile Fasern.

Die kationischen Tenside (C) sind bevorzugt in einer Menge von 0,5 bis 5 Gew.-% enthalten.

Bevorzugt geeignete kationische Tenside zur Ausführung der Erfindung sind quartäre Ammoniumsalze der Formel I

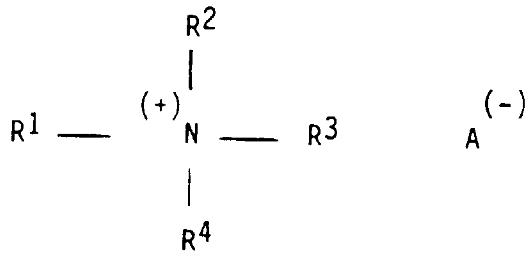

(I),

in der $R^1$ eine lineare oder verzweigte Alkylgruppe mit 12 - 22 C-Atomen oder eine Gruppe $R^5COOC_nH_{2n}$- oder $R^6COONHC_nH_{2n}$-, worin $R^5CO$ und $R^6CO$ lineare Acylgruppen mit 12 - 22 C-Atomen und n = 2 oder 3 ist, $R^2$ und $R^3$ unabhängig voneinander eine der Bedeutungen von $R^1$ haben oder eine Alkylgruppe mit 1 - 4 C-Atomen ist, und $R^4$ eine Alkylgruppe mit 1 - 4 C-Atomen oder eine Hydroxyalkylgruppe mit 2 - 4 C-Atomen ist, und $A^{(-)}$ ein Halogenid- oder Methosulfat-Anion ist. Beispiele für geeignete wasserlösliche kationische Tenside sind z.B. Cetyl-trimethylammoniumchlorid oder N-(2-Hydroxyhexadecyl)-N-(2-hydroxyethyl)-dimethylammonium-chlorid, kationische Derivate von Proteinhydrolysaten, z.B. Lauryldimonium-hydroxypropyl-hydrolized-collagen (Lamequat$^{(R)}$L) oder Gluadin$^{(R)}$WQ.

Als wasserunlösliche kationische Tenside eignen sich bevorzugt die gut biologisch abbaubaren Di-(Fettacyloxyethyl)-hydroxyethyl-methylammonium-salze.

Als wasserlösliche Elektrolytsalze (D) sind in den erfindungsgemäßen Dispersionen alle in Wasser stark dissoziierten und leicht löslichen anorganischen und organischen Salze geeignet. Die Wasserlöslichkeit sollte dabei so hoch sein, daß bei Normaltemperatur (20°C) wenigstens 1 Gew.-% des Salzes in der wäßrigen Phase gelöst ist. Bevorzugt geeignet sind Alkali- oder Erdalkalisalze, z.B. Natriumchlorid, Natriumsulfat, Kaliumchlorid, Kaliumhydrogensulfat, Magnesiumchlorid und Calciumchlorid.

Die Herstellung der erfindungsgemäßen wäßrigen Dispersionen kann ganz einfach durch Erwärmen einer Mischung der Komponenten über die Phaseninversionstemperatur, starke mechanische Vermischung, z.B. Rühren oder Dispergieren und Abkühlen der gebildeten Dispersion bis auf Normaltemperatur (20°C) erfolgen.

Eine bevorzugte Herstellmethode besteht darin, daß man Wachskomponente, Emulgatoren und Tenside auf die Herstelltemperatur aufheizt und mit der ebenfalls auf Herstelltemperatur aufgeheizten wäßrigen Lösung des Elektrolytsalzes mischt. Dabei muß nicht das ganze Wasser eingesetzt werden, sondern nur so viel, wie zur Lösung des Elektrolytsalzes erforderlich ist. Das restliche Wasser kann nach dem Abkühlen der Dispersion kalt zugesetzt werden.

Die Herstelltemperatur sollte oberhalb des Phaseninversionstemperaturbereichs liegen. Diesen Temperaturbereich sollte man daher zunächst anhand eines Probeansatzes ermitteln. Dabei werden alle Komponenten der Emulsion, einschließlich des Wassers und des Elektrolytsalzes erhitzt, wobei man mit Hilfe eines Leitfähigkeitsmeßgerätes den Temperaturbereich bestimmt, bei dem - infolge Phasenumkehr - die Leitfähigkeit stark abnimmt.

Die erfindungsgemäßen kationischen Wachsdispersionen eignen sich für viele interessante technische Anwendungen, insbesondere als Haut- und Haarpflegeemulsionen oder Textilavivagemittel. Um die erfindungsgemäßen Dispersionen für die jeweilige Anwendung geeignet zu machen, können weitere wasserlösliche oder öllösliche Komponenten eingearbeitet werden, die dem Aussehen, der Konservierung, dem Schutz vor Oxidation oder Alterung oder der besseren Handhabung dienen.

Solche an sich üblichen Zusätze sind insbesondere Farbstoffe, optische Aufheller, antimikrobielle Stoffe, Antioxidantien, wasserlösliche Verdickungsmittel, z.B. nichtionische oder kationische Polymere oder Celluloseether, oder auch wasserunlösliche Verdickungsmittel, wie z.B. Schichtsilikate oder feinteilige Kieselsäuren.

Die folgenden Beispiele sollen den Gegenstand der Erfindung näher erläutern:

## Beispiele

Die folgenden Dispersionen gemäß Beispiel 1 - 7 wurden wie folgt hergestellt:

Wachskomponente, Emulgatoren, Tenside und die wäßrige Lösung der Elektrolyte wurden gemischt und auf die Herstelltemperatur aufgeheizt. Da die Phaseninversionstemperatur infolge des Elektrolytzusatzes unter 100°C lag, wurde als Herstelltemperatur 96,5°C bzw. 98,5°C gewählt (vgl. Tabelle).

Die Vergleichsdispersion, Beispiel 7V, wurde bei 65°C (unterhalb der Phaseninversionstemperatur) hergestellt. Die Dispersionen 1 - 7 waren dünnflüssig und schimmerten bläulich in der Durchsicht. Die Dispersion 7V war weiß und dickflüssig.

In der folgenden Tabelle sind die Zusammensetzungen der Beispiele, die Phaseninversionstemperatur (PIT) und für das Beispiel 7 und 7V die mittlere Teilchengröße angegeben.

Die mittlere Teilchengröße wurde aus der Teilchengrößenverteilung, gemessen durch dynamische Lichtstreuung sowie mikroskopisch mit automatischer Bildverarbeitung, bestimmt.

Zur Herstellung der Beispiele wurden die folgenden Handelsprodukte eingesetzt:

| | |
|---|---|
| Mergital B10: | Behenylalkohol-poly-(10EO)-glycolether |
| Eumulgin B1: | Cetyl-/Stearylalkohol-poly-(10E0)-glycolether (Ceteareth 12) |
| Cutina GMS: | Glycerinmono/di-stearat |
| Cutina CP: | Cetylpalmitat |
| Cetiol S: | 1,3-Di-(2-ethylhexyl)-cyclohexan |
| Dehyquart A: | Cetyl-trimethyl-ammoniumchlorid (30 %ige Lösung) |
| Dehyquart E: | N-(2-Hydroxyhexadecyl)-N-(2-hydroxyethyl)-dimethyl-ammoniumchlorid (30 %ige Lösung) |

Dehyquart AU56:

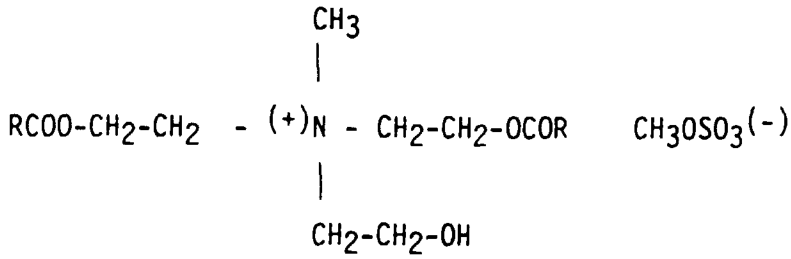

RCO = Palmöl-fettacylrest

Tabelle

| Komponenten (Gew.-%) | 1 | 2 | 3 | 4 | 5 | 6 | 7 | 7V |
|---|---|---|---|---|---|---|---|---|
| Cutina CP | 35,0 | 30,0 | 25,0 | 35,0 | 35,0 | 35,0 | 35,0 | 35,0 |
| Cetiol S | - | 5,0 | 10,0 | - | - | - | - | - |
| Mergital B10 | 6,5 | 5,0 | 5,0 | 6,1 | - | 5,0 | 5,0 | 5,0 |
| Eumulgin B1 | - | - | - | - | 5,8 | - | - | - |
| Cutina GMS | 3,0 | 4,0 | 4,0 | 1,9 | 2,2 | 4,0 | 4,0 | 4,0 |
| Dehyquart A | 0,5 | 1,0 | 1,0 | - | 2,0 | - | 1,0 | 1.0 |
| Dehyquart E | - | - | - | - | - | 1,0 | - | - |
| Dehyquart AU56 | - | - | - | 2,0 | - | - | - | - |
| 4 %ige Lösung von NaCl in Wasser | 55,0 | 55,0 | 55,0 | 55,0 | 55,0 | 55,0 | 55,0 | 55,0 |
| Herstellung (°C) | 96,5 | 96,5 | 96,5 | 96,5 | 96,5 | 96,5 | 98,5 | 65 |
| PIT (°C) | 83 | 83 | 71 | 74 | 48 | 90 | 95 | 95 |
| mittlerer Teilchendurchmesser (nm) | | | | | | | 150 ±50 | 8000 ±3000 |

**Patentansprüche**

1. Wäßrige Dispersionen wachsartiger Fettstoffe und Paraffine mit einem mittleren Teilchendurchmesser von 100 bis 500 nm, dadurch gekennzeichnet, daß sie

   (A) 20 bis 50 Gew.-% dispergierte wachsartige bei Normaltemperatur (20°C) feste und in einem Bereich von 40 - 90°C schmelzbare Fettstoffe oder Paraffine

   (B) 1 bis 15 Gew.-% nichtionogene Emulgatoren

   (C) 0,5 bis 40 Gew.-% kationische Tenside und

   (D) 1 bis 5 Gew.-% wasserlösliche Alkali- oder Erdalkalisalze, die bei Normaltemperatur (20°C) zu wenigstens 1 Gew.-% in der wäßrigen Phase gelöst sind,

   enthalten.

2. Wäßrige Dispersionen nach Anspruch 1, dadurch gekennzeichnet, daß die wachsartigen Fettstoffe (A) Ester aus Fettsäuren mit 12 - 22 C-Atomen und Fettalkoholen mit 12 - 22 C-Atomen sind.

3. Wäßrige Dispersion nach Anspruch 1 - 2, dadurch gekennzeichnet, daß als nichtionogener Emulgator (B) eine

Kombination aus

(B1) einem hydrophilen nichtionischen Emulgator mit einem HLB-Wert von 11 bis 15 und

(B2) einen lipophilen Coemulgator aus der Gruppe der gesättigten Fettalkohole mit 16 bis 22 C-Atomen und Partialestern von Polyolen mit 2 - 6 C-Atomen und Fettsäuren mit 16 - 22 C-Atomen,

im Gewichtsverhältnis von (B1) : (B2) = 1 : 1 bis 5 : 1 enthalten ist.

**4.** Wäßrige Dispersionen nach Anspruch 1 - 3, dadurch gekennzeichnet, daß als kationische Tenside (C) quartäre Ammoniumsalze der Formel I

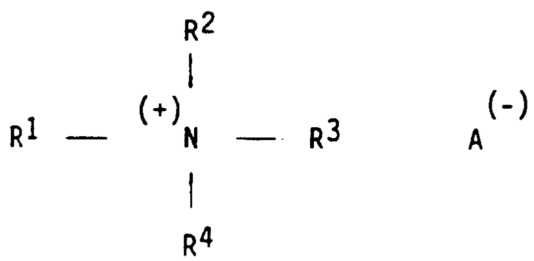

(I),

enthalten sind, in der $R^1$ eine lineare oder verzweigte Alkylgruppe mit 12 - 22 C-Atomen oder eine Gruppe $R^5COOC_nH_{2n}$- oder $R^6$-$COONHC_nH_{2n}$-, worin $R^5CO$ und $R^6CO$ lineare Acylgruppen mit 12 - 22 C-Atomen und n = 2 oder 3 ist, $R^2$ und $R^3$ unabhängig voneinander eine der Bedeutungen von $R^1$ hat oder eine Alkylgruppe mit 1 - 4 C-Atomen ist und $R^4$ eine Alkylgruppe mit 1 - 4 C-Atomen oder eine Hydroxyalkylgruppe mit 2 - 4 C-Atomen ist und A(-) ein Halogenid- oder Methosulfat-Anion ist.

## Claims

**1.** Aqueous dispersions of wax-like fatty compounds and paraffins with a mean particle diameter of 100 to 500 nm, characterized in that they contain

(A) 20 to 50% by weight of dispersed wax-like fatty compounds or paraffins which are solid at normal temperature (20°C) and can be melted at a temperature of 40 to 90°C,
(B) 1 to 15% by weight of nonionic emulsifiers,
(C) 0.5 to 40% by weight of cationic surfactants and
(D) 1 to 5% by weight of water-soluble alkali metal or alkaline earth metal salts of which at least 1% by weight is dissolved in the aqueous phase at normal temperature (20°C).

**2.** Aqueous dispersions as claimed in claim 1, characterized in that the wax-like fatty compounds (A) are esters of $C_{12-22}$ fatty acids and $C_{12-22}$ fatty alcohols.

**3.** Aqueous dispersions as claimed in claims 1 and 2, characterized in that the nonionic emulsifier (B) is a combination of

(B1) a hydrophilic nonionic emulsifier with an HLB value of 11 to 15 and
(B2) a lipophilic co-emulsifier from the group of saturated $C_{16-22}$ fatty alcohols and partial esters of $C_{2-6}$ polyols and $C_{16-22}$ fatty acids

in a ratio by weight of (B1) to (B2) of 1:1 to 5:1.

**4.** Aqueous dispersions as claimed in claims 1 to 3, characterized in that the cationic surfactants (C) are quaternary ammonium salts corresponding to formula (I):

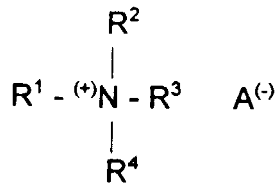

(I)

in which $R^1$ is a linear or branched $C_{12-22}$ alkyl group or a group $R^5COOC_nH_{2n}$ - or $R^6COONHC_nH_{2n}$-, where $R^5CO$ and $R^6CO$ are linear $C_{12-22}$ acyl groups and n = 2 or 3, $R^2$ and $R^3$ independently of one another have one of the meanings of $R^1$ or represent a $C_{1-4}$ alkyl group and $R^4$ is a $C_{1-4}$ alkyl group or a $C_{2-4}$ hydroxyalkyl group and $A^{(-)}$ is a halide or methosulfate anion.

**Revendications**

1. Dispersions aqueuses de paraffines et de matières grasses cireuses, possédant un diamètre de particule moyen de 100 à 500 nm, caractérisées en ce qu'elles renferment:

   (A) 20 à 50 % en poids de paraffines ou de matières grasses cireuses, dispersées, solides à température ambiante (20 °C) et fusibles dans une plage de 40 à 90 °C

   (B) 1 à 15 % en poids d'émulsionnants non ioniques

   (C) 0,5 à 40 % en poids de tensioactifs cationiques et

   (D) 1 à 5 % en poids de sels de métaux alcalins ou alcalino-terreux électrolytiques solubles dans l'eau, qui sont dissous à au moins 1 % en poids dans la phase aqueuse à température normale (20 °C).

2. Dispersions aqueuses selon la revendication 1, caractérisées en ce que les matières grasses cireuses (A) sont des esters d'acides gras comportant 12 à 22 atomes de C et d'alcools gras possédant 12 à 22 atomes de C.

3. Dispersion aqueuse selon la revendication 1-2, caractérisée en ce qu'elle contient comme émulsionnant non ionique (b), une association

   (B1) d'un émulsionnant non ionique hydrophile présentant une valeur HLB de 11 à 15 et
   (B2) d'un co-émulsionnant lipophile appartenant au groupe des alcools gras saturés comportant 15 à 22 atomes de C et des esters partiels de polyols possédant 2 à 6 atomes de C et d'acides gras présentant 16 à 22 atomes de C,

   dans un rapport pondéral de (B1):(B2) égal à 1:1 à 5:1.

4. Dispersions aqueues selon la revendication 1-3, caractérisées en ce qu'elles renferment comme tensioactifs cationiques (C), des sels quaternaires d'ammonium de la formule I

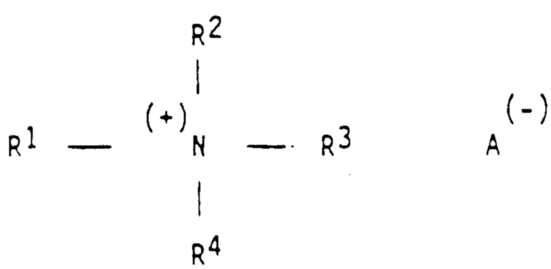

(I),

   dans laquelle $R^1$ représente un groupe alkyle linéaire ou ramifié comportant 12 à 22 atomes de C ou un groupe

$R^5COOC_nH_{2n}$- ou un groupe $R^5COONHC_nH_{2n}$-, dans lesquels $R^5CO$ et $R^6CO$ correspondent à des groupes acyle linéaires comportant 12 à 22 atomes de C et n = 2 ou 3, $R^2$ et $R^3$ possèdent indépendamment l'un de l'autre une des significations de $R^1$ ou sont un groupe alkyle comportant 1 à 4 atomes de C, et $R^4$ représente un groupe alkyle possédant 1 à 4 atomes de C ou un groupe hydroxyalkyle présentant 2 à 4 atomes de C, et $A^{(-)}$ est un anion d'halogénure ou de méthosulfate.